(19) Europäisches Patentamt
European Patent Office
Office européen des brevets

(11) Publication number: **0 355 169 A1**

(12) # EUROPEAN PATENT APPLICATION published in accordance with Art. 158(3) EPC

(21) Application number: 89901747.9

(51) Int. Cl.[5]: A61K 31/495 , A61K 31/505

(22) Date of filing: 27.01.89

(86) International application number: PCT/JP89/00078

(87) International publication number: WO 89/06961 (10.08.89 89/18)

(30) Priority: 29.01.88 JP 19055/88

(43) Date of publication of application: 28.02.90 Bulletin 90/09

(84) Designated Contracting States: AT CH DE FR GB IT LI LU NL SE

(71) Applicant: MITSUBISHI KASEI CORPORATION
5-2, Marunouchi 2-chome Chiyoda-ku
Tokyo 100(JP)

(72) Inventor: SAITO, Ken-Ichi
10-4-15-301, Ogawa 2-chome
Machida-shi Tokyo 194(JP)
Inventor: TOBE, Akihiro
B-211 Mitsubishi Kasei Sakuradai Apt 3, Sakuradai
Midori-ku Yokohama-shi Kanagawa 227(JP)
Inventor: IWATA, Heitaro
6-41, Yamatedai 6-chome
Ibaraki-shi Osaka 567(JP)
Inventor: BABA, Akemichi
17-12-617, Mondoso
Nishinomiya-shi Hyogo 662(JP)
Inventor: MATSUDA, Toshio
13-11-605, Senrioka Higashi 1-chome
Settsu-shi Osaka 566(JP)

(74) Representative: Vossius & Partner
Siebertstrasse 4 P.O. Box 86 07 67
D-8000 München 86(DE)

(54) **ANTIANXIETY DRUG.**

(57) An antianxiety drug containing as an active ingredient a piperazine derivative represented by general formula (1) (wherein m represents an integer of 2, 3 or 4, X represents -O-, (a) (wherein 1 = 0 or 1), (b), (c), (d) or (e), Ar represents a pyridyl group, a pyrimidinyl group or a phenyl group optionally substituted by halogen, trifluoromethyl, alkoxy or alkyl, $R_1$, $R_2$ and $R_3$ may be the same or different and each represents a lower alkoxy group, or $R_3$ represents a hydrogen atom and $R_1$ and $R_2$ are taken together to form (f) (wherein n = 1, 2 or 3)) or an acid addition salt thereof is disclosed. The compounds have a high ability of binding with a $5\text{-HT}_{1A}$ receptor, one of 5-hydroxytriptamine(5-HT) receptors, in vivo, thus showing an antianxiety effect.

EP 0 355 169 A1

Xerox Copy Centre

DESCRIPTION

Title of the Invention:

Anxiolytic Drug

Field of the Invention:

The present invention relates to an anxiolytic drug containing as an active ingredient a specified piperazine derivative and its acid addition salt.

Background of the Invention:

Benzodiazepine compounds have conventionally been known as anxiolytic drugs. Recently, Buspirone [N-(4-(4-(2-pyrimidinyl)-1-piperazinyl)butyl)-1,1-cyclopentanediacetamide hydrochloride] and other compounds have become recognized as new anxiolytic drugs with action mechanisms different from those of the benzodiazepines, but the advent of newer anxiolytic drugs is being desired.

Taking notice of the piperazine derivatives, the present inventors earnestly investigated to develop compounds which may be used as anxiolytic drugs. A variety of piperazine derivatives have been recognized to have a satisfactory antihypertensive effect (for example, Japanese Patent Application Laying-Open (KOKAI) No. 57-80379, No. 57-114588, No. 58-24563 and No. 58-154573, but were not known to have anxiolytic activities.

Now, the present inventors have discovered that certain piperazine derivatives and their acid addition salts have satisfactory anxiolytic effects, and thus the present invention has been completed.

Disclosure of the Invention:

The present invention relates to an anxiolytic drug containing as an active ingredient a piperazine derivative represented by the following general formula (I):

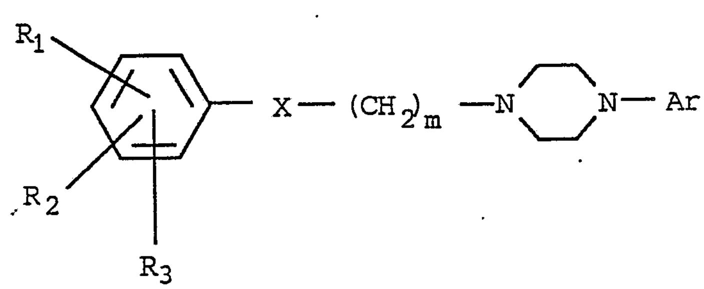

..... (I)

wherein

m represents an integer from 2 to 4,

X represents –O–, $-\underset{(O)_\ell}{S}-$ ($\ell$ = 0 or 1), $-\underset{H}{N}-\underset{\|O}{C}-$, $-\underset{H}{N}-$, $-\underset{\|O}{C}-$

or $-\overset{H}{\underset{OH}{C}}-$,

Ar represents a pyridyl group, a pyrimidinyl group, or a phenyl group which may be substituted with halogen atom, trifluoromethyl group, alkoxy group or alkyl group, and

$R_1$, $R_2$ and $R_3$ which may be identical or different represent lower alkoxy groups, or $R_3$ is a hydrogen atom and $R_1$ when taken together with $R_2$ forms 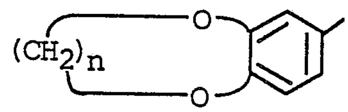

(n = 1, 2 or 3)

and its acid addition salt.

The present invention will be described in detail below. The piperazine derivatives used in the present invention are those represented by the above general formula (I).

The suitable compounds in the present invention are those represented by the above formula (I) wherein m represents 3, X represents -O-, -CO- or -CH(OH)-, Ar represents a pyridyl group or a phenyl group which may be substituted with any one of the above substituents, and $R_1$, $R_2$ and $R_3$ which may be identical or different represent $C_1$-$C_3$ lower alkoxy groups, or $R_3$ is a hydrogen atom and $R_1$ when taken together with $R_2$ forms 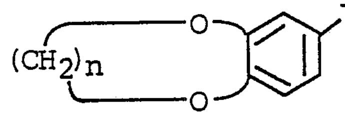

(n = 1 or 2); and its acid addition salt.

The acid addition salt of the present invention may be formed with any inorganic acid such as hydrochloric acid, hydrobromic acid, sulfuric acid, phosphoric acid and nitric acid, or any organic acid such as acetic acid, succinic acid, adipic acid, propionic acid, tartaric acid, fumaric acid, maleic acid, oxalic acid, citric acid, benzoic acid, toluenesulfonic acid and methanesulfonic acid.

The above compounds may be easily synthesized, for example in accordance with the procedures described in Japanese Patent Application Laying-Open (KOKAI) No. 57-80379, No. 57-114588, No. 58-24563 and No. 58-154573.

The compounds of the present invention bind to 5-$HT_{1A}$ receptor which is one of the receptors for 5-hydroxytryptamine, as shown in the following Examples.

Compounds which bind to 5-$HT_{1A}$ receptor, for example, Buspirone [N-(4-(4-(2-pyrimidinyl)-1-piperazinyl)-butyl)-1,1-cyclopentadiacetamide hydrochloride] (Naunyn-Schmiedeberg's Arch. Pharmakol., 328, 467, 1985) and Ipsapirone [2-(4-(4-(2-pyrimidinyl)-1-piperazinyl)butyl)-1,2-benzisothiazol-3-(2H)one-1,1-dioxydehydrochloride] and SM-3997 [3aα, 4β, 7β, 7aα-hexahydro-2-(4-(4-(2-pyrimidinyl)-1-piperazinyl)butyl)-4,7-metano-1H-isoindole-1,3(2H)-dione dihydrogen citrate] (Naunyn-Schmiedeberg's Arch. Pharmakol., 328, 467, 1985; Japan. J. Pharmacol., 45, 493, 1987) have been known to exert an anxiolytic effect. The compounds of the present invention may be used as anxiolytic drugs based on their similar activities.

When intended for use as anxiolytic drugs, the compounds of the present invention may be administered through any routes. But, it is preferable to administer the anxiolytic drugs of the present invention

parenterally including subcutaneous, intravenous, intramuscular or intraperitoneal injection, or orally.

The dosage is determined depending on age, health condition and body weight of the patient, type and frequency of combined treatment, if any as well as nature of desired effects, and so on.

Generally, the daily dosage of the active ingredient is 0.01 to 10.0 mg per kg body weight, usually 0.1 to 3 mg/kg body weight, in a single or divided administration.

For oral administration, the compounds of the present invention are used in the form such as tablets, capsules, powder, liquid, or elixir. And, for parenteral administration, they are used as sterilized liquids including suspensions. Solid or liquid, nontoxic pharmaceutical carriers may be included in the preparations of the present invention when used in any of the above dosage forms.

As an example of solid carrier, usual gelatin type capsules are used. And, the active ingredient may be formed into tablets or powder in combination with or without any additives.

These capsules, tablets and powder generally contain 5 to 95% by weight, preferably 25 to 90% by weight of the active ingredient.

That is, the preparations provided by the present invention advisably contain 5 to 500 mg, preferably 25 to 250 mg of the active ingredient for administration in these dosage forms.

As liquid carriers, water, oils of animal or plant origin such as petroleum oil, peanut oil, soybean oil, mineral oil and sesame oil, or synthetic oils may be used.

Furthermore, physiological saline solution, dextrose or similar sucrose solution, and glycols such as ethylene glycol, propylene glycol and polyethylene glycol are generally preferable as liquid carriers. Particularly in case of injection employing physiological saline, the injection is to be formulated so that the content of the active ingredient is usually 0.5 to 20% by weight, preferably 1 to 10% by weight.

Liquid preparations for oral administration may advisably be in the form of suspension or syrup containing 0.5 to 10% by weight of the active ingredient.

On these preparations, flavoring agents and aqueous excipients such as syrup and pharmaceutical micelles may be used as carriers.

<u>Preferred Embodiments of the Invention</u>:

The present invention will now be illustrated by the following examples, but it is to be clearly understood

that the description made in connection with these examples is only for the purpose of illustration and not as a limitation on the scope of invention.

Example 1

The compounds of the present invention listed in Table 1 were synthesized by the conventional techniques and their affinities for 5-$HT_{1A}$ receptor were determined by a binding assay using 8-hydroxy-2-(di-n-propylamino) tetralin ([3H]8-OH-DPAT) which is a selective ligand for 5-$HT_{1A}$ receptor (Neuropharmacol., 26, 139, 1987). To say more exactly, a rat brain was homogenized in Tris buffer and centrifuged. The resultant sediment was rehomogenized using Tris buffer and incubated at 37°C for 10 minutes, followed by centrifugation. The resulting deposit was homogenized in Tris buffer containing pargyline, calcium chloride and ascorbic acid and subjected to binding assay (membrane preparation).

The assay was carried out by combining the membrane preparation with [3H]8-OH-DPAT and the test compound and then incubating at 37°C for 10 minutes.

Subsequently, the mixture was immediately filtered through a Whatman GF/B filter and the radio-activity remaining on the filter was measured by liquid chromatography.

The binding capacity of the test compound to 5-$HT_{1A}$ receptor was expressed as Ki value calculated by the formula:

$$Ki = \frac{IC_{50}}{1 + \frac{[L]}{kd}}$$

wherein [L] denotes the concentration of [3H]8-OH-DPAT, kd represents the dissociation constant, and $IC_{50}$ represents the concentration of the test compound required to produce a 50% inhibition of [3H]8-OH-DPAT binding. It is considered, therefore, that the lower the Ki value, the greater the potential usefulness of the test compound as an anxiolytic drug.

Results are shown in Table 1.

Table 1

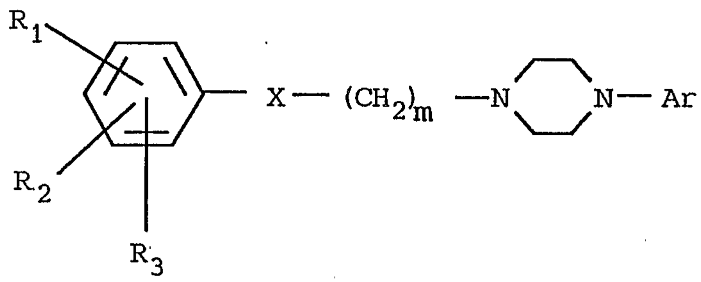

| No. | m | $-R_1, -R_2$ | $-R_3$ | Substituted positions | X | -Ar | Addition salt | Ki value (nM) |
|---|---|---|---|---|---|---|---|---|
| 1 | 3 | 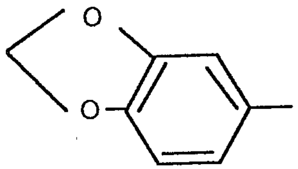 | H | — | -O- | 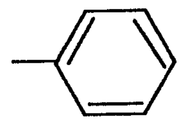 | 2HCℓ | 4.7 |
| 2 | 3 | 〃 | 〃 | 〃 | -O- | 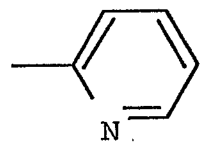 | 3HCℓ | 2.4 |
| 3 | 3 | 〃 | 〃 | 〃 | -O- | 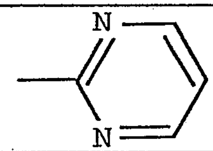 | 4HCℓ | 10.1 |
| 4 | 4 | 〃 | 〃 | 〃 | -O- | 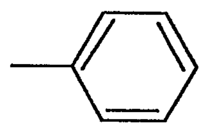 | 2HCℓ | 10.1 |
| 5 | 4 | 〃 | 〃 | 〃 | -O- | 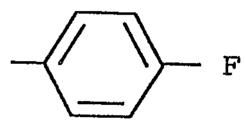 | 2HCℓ | 33.2 |

| No. | m | -$R_1$, -$R_2$ | -$R_3$ | Substituted positions | X | -Ar | Addition salt | Ki value (nM) |
|---|---|---|---|---|---|---|---|---|
| 6 | 4 | 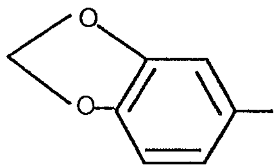 | H | — | -O- | 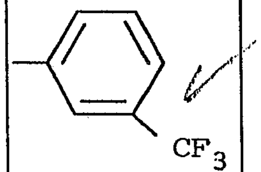 | 2HCℓ | 55.9 |
| 7 | 3 | $CH_3O-$, $CH_3O-$ | $CH_3O-$ | 3,4,5 | -O- | 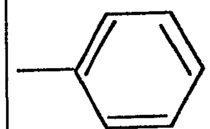 | HCℓ | 5.7 |
| 8 | 3 | $CH_3O-$, $CH_3O-$ | H | 3,4 | -O- | 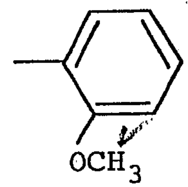 | 2HCℓ | 14.3 |
| 9 | 3 | $CH_3O-$, $CH_3O-$ | 〃 | 1,5 | -O- | 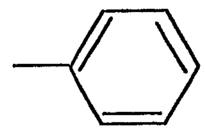 | 2HCℓ | 63.7 |
| 10 | 2 | 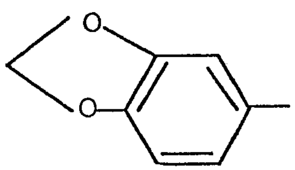 | 〃 | — | -NC-<br>H ‖<br>O | 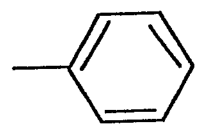 | — | 25.4 |
| 11 | 3 | 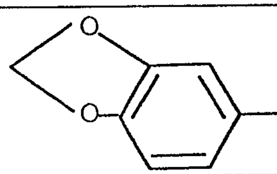 | 〃 | — | -N-<br>H | 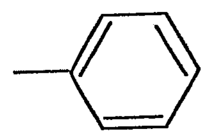 | 3HCℓ | 18.0 |

| No. | m | -$R_1$, -$R_2$ | -$R_3$ | Substituted positions | X | -Ar | Addition salt | Ki value (nM) |
|---|---|---|---|---|---|---|---|---|
| 12 | 3 | 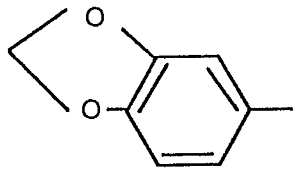 | H | — | -O- | 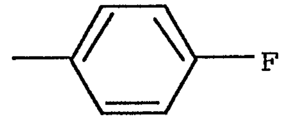 | 2HCℓ | 16.0 |
| 13 | 3 | 〃 | 〃 | 〃 | -O- | 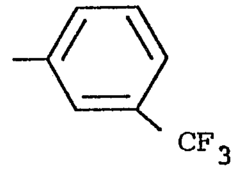 | 2HCℓ | 8.0 |
| 14 | 3 | 〃 | 〃 | 〃 | -O- | 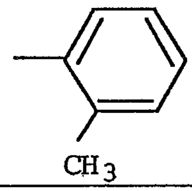 | 2HCℓ | 101.0 |
| 15 | 3 | 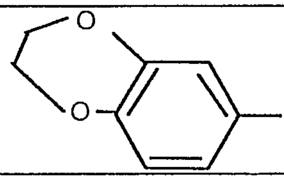 | 〃 | 〃 | -O- | 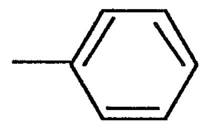 | 4HCℓ | 5.7 |
| 16 | 2 | 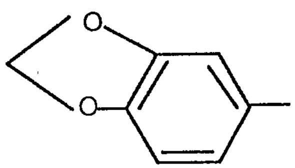 | 〃 | 〃 | -O- | 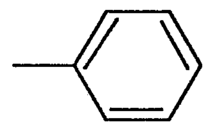 | 2HCℓ | 127.2 |
| 17 | 3 | 〃 | 〃 | 〃 | 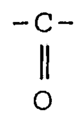 | 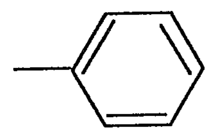 | 2HCℓ | 5.7 |

| No. | m | -$R_1$, -$R_2$ | -$R_3$ | Substituted positions | X | -Ar | Addition salt | Ki value (nM) |
|---|---|---|---|---|---|---|---|---|
| 18 | 3 | 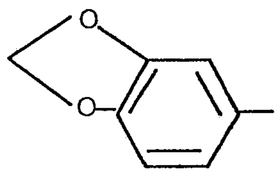 | H | — | 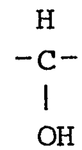 | 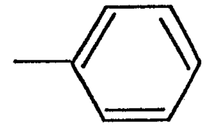 | 2HCℓ | 8.0 |
| 19 | 4 | 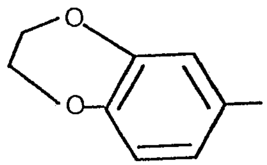 | 〃 | 〃 | -O- | 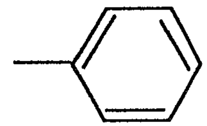 | 2HCℓ | 16.0 |
| 20 | 4 | 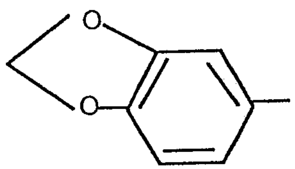 | 〃 | 〃 | 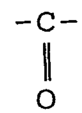 | 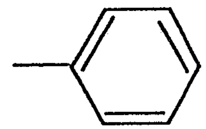 | 2HCℓ | 10.1 |
| 21 | 3 | 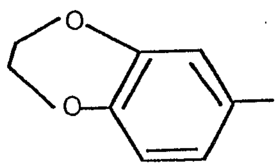 | 〃 | 〃 | -S- | 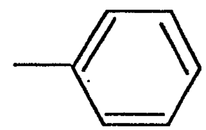 | — | 71.2 |
| 22 | 3 | 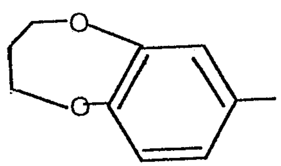 | 〃 | 〃 | 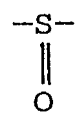 | 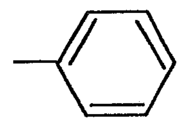 | — | 25.4 |
| Reference | | Buspirone | | | | | | 14 |
| Reference | | Ipsapirone | | | | | | 2.2 |

Availability in Industry:

The compounds of the present invention have proven to have a binding capacity to $5\text{-}HT_{1A}$ comparable to or even greater than that of the known anxiolytic drugs, Buspirone and Ipsapirone, and accordingly, may be used as anxiolytic drugs based on the same action as that of the known drugs.

Furthermore, the compounds of the present invention may have marked usefulness as anxiolytic drugs with a high degree of safety because they are less liable to cause the side effects such as drowsiness and muscle relaxation which are observed in the use of the benzodiazepine compounds which are conventional anxiolytic drugs.

## CLAIMS

1. An anxiolytic drug containing as an active ingredient a piperazine derivative represented by the following general formula (I):

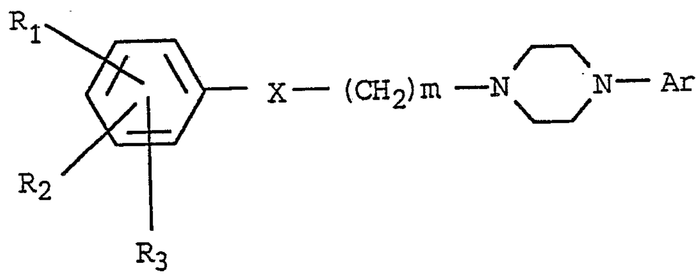

..... (I)

wherein

m represents an integer from 2 to 4,

X represents -O-, 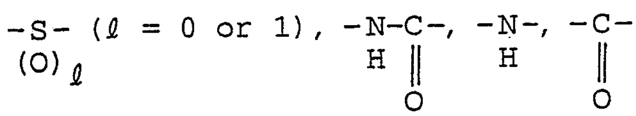 ($\ell$ = 0 or 1), , ,

or 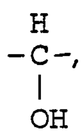,

Ar represents a pyridyl group, a pyrimidinyl group, or a phenyl group which may be substituted with halogen atom, trifluoromethyl group, alkoxy group or alkyl group, and

$R_1$, $R_2$ and $R_3$ which may be identical or different represent lower alkoxy groups, or $R_3$ is a hydrogen atom and $R_1$ when taken together with $R_2$ forms 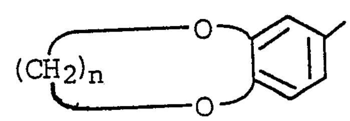

(n = 1, 2 or 3);

and its acid addition salt.

2. The anxiolytic drug according to claim 1, which contains as an active ingredient a piperazine derivative of the general formula (I) wherein $R_1$ when taken together with $R_2$ forms the formula 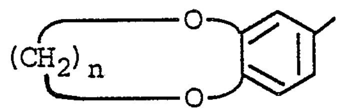 (n is 1, 2 or 3) and $R_3$ is a hydrogen atom, or its acid addition salt.

# INTERNATIONAL SEARCH REPORT

International Application No PCT/JP89/00078

**I. CLASSIFICATION OF SUBJECT MATTER** (if several classification symbols apply, indicate all) [6]

According to International Patent Classification (IPC) or to both National Classification and IPC

Int. $Cl^4$ A61K31/495, A61K31/505

**II. FIELDS SEARCHED**

Minimum Documentation Searched [7]

| Classification System | Classification Symbols |
|---|---|
| IPC | C07D31/495, C07D31/505 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | GB, A, 1166595 (Byk-Gulden Lomberg Chemische Fabrik G.m.b.H.) 8 October 1969 (08. 10. 69) (Family: none) | 1-2 |
| A | JP, A, 58-192824 (Mitsubishi Chemical Industries Ltd.) 10 November 1983 (10. 11. 83) Claim (Family: none) | 1-2 |
| A | JP, A, 59-7120 (Mitsubishi Chemical Industries Ltd.) 14 January 1984 (14. 01. 84) Claim (Family: none) | 1-2 |
| A | JP, A, 59-10517 (Mitsubishi Chemical Industries Ltd.) 20 January 1984 (20. 01. 84) Claim (Family: none) | 1-2 |

* Special categories of cited documents: [10]

- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step
- "Y" document of particular relevance; the claimed invention cannot be considered to involve an inventive step when the document is combined with one or more other such documents, such combination being obvious to a person skilled in the art
- "&" document member of the same patent family

**IV. CERTIFICATION**

| Date of the Actual Completion of the International Search | Date of Mailing of this International Search Report |
|---|---|
| April 7, 1989 (07. 04. 89) | April 24, 1989 (24. 04. 89) |
| **International Searching Authority** | **Signature of Authorized Officer** |
| Japanese Patent Office | |

FURTHER INFORMATION CONTINUED FROM THE SECOND SHEET

| | | |
|---|---|---|
| A | JP, A, 59-152379 (Recordati, S.A., Chemical & Pharmaceutical Co.) 31 August 1984 (31. 08. 84) Claim & EP, A1, 120558 & AU, A1, 2449484 & DK, A, 48584 & ES, A1, 529642 | 1-2 |
| A | "J. Med. Chem.", 11(6) p. 1151-5, Pharmcol. Res. Div., Sci. Union Assoc. | 1-2 |

V.☐ OBSERVATIONS WHERE CERTAIN CLAIMS WERE FOUND UNSEARCHABLE [1]

This international search report has not been established in respect of certain claims under Article 17(2) (a) for the following reasons:

1.☐ Claim numbers ..........., because they relate to subject matter not required to be searched by this Authority, namely:

2.☐ Claim numbers ......... .., because they relate to parts of the international application that do not comply with the prescribed requirements to such an extent that no meaningful international search can be carried out, specifically:

3.☐ Claim numbers ............, because they are dependent claims and are not drafted in accordance with the second and third sentences of PCT Rule 6.4(a).

VI.☐ OBSERVATIONS WHERE UNITY OF INVENTION IS LACKING [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.